# EUROPEAN PATENT APPLICATION

(11) **EP 3 875 116 A1**
(43) Date of publication of application: **08.09.2021**
(21) Application number: 19877954.8
(22) Date of filing: 01.11.2019
(51) Int. Cl.: A61K 45/00, A61K 9/08, A61K 9/10, A61K 9/107, A61K 9/14, A61K 9/16, A61K 9/20, A61K 9/48, A61K 31/4436, A61K 38/12, A61P 19/02, A61P 29/00

(54) **THERAPEUTIC AGENT FOR ARTHRITIS**

(30) Priority: 02.11.2018 JP 2018207209
(71) Applicant: Osaka University, Suita-shi, Osaka 565-0871 (JP)
(72) Inventor: ISHII, Masaru, Suita-shi, Osaka 565-0871 (JP); KIKUTA, Junichi, Suita-shi, Osaka 565-0871 (JP); HASEGAWA, Tetsuo, Suita-shi, Osaka 565-0871 (JP)
(74) Representative: Hoffmann Eitle
(86) International application number: PCT/JP2019/043057
(87) International publication number: WO 2020/091052

(57) **Abstract**

The present invention provides with a therapeutic agent for arthritis, which contains a low-molecular-weight FoxM1 inhibitor, e.g., thiostrepton, as an active ingredient. FoxM1 is expressed in an osteoclast progenitor that occurs specifically in an inflamed synovium in arthritis, and is not involved in the function of an osteoclast that is present in the bone marrow in a normal person and is involved in normal bone metabolism. Therefore, arthritis can be prevented and/or treated without any adverse side effect by using a FozMl inhibitor.

## Description

### Technical Field

The present invention relates to a new therapeutic agent for arthritis. The present invention relates more specifically to a therapeutic agent for arthritis containing an inhibitor of the transcription factor FoxM1 as an active ingredient and preferably to a therapeutic agent for rheumatoid arthritis.

### Background Art

Arthritis is a general term for illnesses accompanied with the inflammation of articulations. In addition to rheumatoid arthritis, for example, spondylarthritis, histiocytosis, and the like are mentioned as chronic inflammatory diseases resulting in morbid bone destruction.

Especially, rheumatoid arthritis is a type of collagen disease, and immunomodulators, immunosuppressants, or biological preparations have been used for treatment thereof. However, it is shown that since these therapeutic agents suppress immunoreaction, the risk of contracting severe infectious diseases increases (the Non Patent Literature 1 described below).

Meanwhile, it is widely known that FoxM1 (Forkhead box M1) is one of the transcription factors which regulate the expression of genes important for cell proliferation, and FoxM1 relates to the control of oncogenesis or cell proliferation. Methods for treating cancer by controlling FoxM1 have also been proposed. For example, an antibody of FoxMl (the Patent Literature 1 described below), the use of the antibody by controlling its expression for treating and/or preventing cancer and/or delaying the progress of cancer (the Patent Literatures 2 and 3 described below), and a method for treating cancer using a compound which inhibits the activity of FoxM1 (the Patent Literature 3 described below) have been proposed.

Besides, there is a report about the FoxM1 inhibitory factor RCM-1, which suppresses the dysplasia of germ cells in a mouse exposed to an allergen and hinders the signal transduction of IL13 and STAT6. The use of a low molecular weight RCM-1 identified here as a therapeutic agent for chronic airway diseases such as asthma has been suggested (the Non Patent Literature 2 described below).

However, it has never been reported until now that FoxM1 is expressed in osteoclast precursor cells described below, and any method for treating arthritis using a FoxM1 inhibitor is not known at all.

### Citation List

### Patent Literature

Patent Literature 1
   Japanese Patent Laid-Open No. 2014-193893
Patent Literature 2
   Japanese Translation of PCT International Application Publication No. 2018-505880
Patent Literature 3
   Japanese Translation of PCT International Application Publication No. 2016-509596

### Non Patent Literature

Non Patent Literature 1
   Singh, J.A., Lancet, 2015
Non Patent Literature 2
   L. Sun et al., Science Signal, 10, eaai8583(2017)

### Summary of Invention

### Technical Problem

Although current immunomodulators, immunosuppressants, or biological preparations used for treatment of collagen disease are intended to control the state of the disease by suppressing the immunity, the immunoreaction to foreign microorganisms is also meanwhile restrained simultaneously, and the risk of contracting severe infectious diseases therefore increases. An object of the present invention is to provide a novel therapeutic agent for arthritis based on a new mechanism without such a side effect.

### Solution to Problem

The present inventors have identified osteoclast precursor cells which appear in inflammatory synovial membranes specifically and revealed that the transcription factor FoxM1 controls the pathogenicity of the cells by research using arthritis model mice. Furthermore, the present inventors have confirmed that, in a rheumatoid arthritis model, a FoxM1 inhibitor suppresses the differentiation into osteoclasts in vitro and articular destruction in vivo, and, also in humans, the osteoclast differentiation of monocyte and macrophage lineage cells collected from a sample of a rheumatoid arthritis patient is suppressed by the inhibitor and completed the present invention.

### Advantageous Effects of Invention

The transcription factor FoxM1 is expressed in osteoclast precursor cells which appear specifically in inflammatory synovial membranes of arthritis, and does not relate to the function of osteoclasts which exist in the bone marrow of healthy persons and relate to the normal bone metabolism. Therefore, the use of the FoxM1 inhibitor enables preventing and/or treating arthritis without side effects.

The use of a therapeutic agent containing the FoxM1 inhibitor as an active ingredient enables avoiding the above-described risk of contracting severe infectious diseases caused by foreign microorganisms. For example, the use of a low molecular weight compound such as thiostrepton as the FoxM1 inhibitor also enables reducing a medical economic burden as compared with treatment with existing biological preparations.

### Brief Description of Drawings

[Figure 1A] Figure 1A shows the results obtained by gating CD45-positive cells of blood and a synovial membrane of an arthritis model mouse by flow cytometry and identifying cell fractions using the fluorescence of CX3CR1-EGFP and Ly6C-APC.
[Figure 1B] Figure 1B shows the difference between the cell surface markers of the osteoclast precursor cells (hereinafter referred to as R3s) existing in an inflammatory synovial membrane and osteoclast precursor cells (BM-OPs) existing in bone marrow.
[Figure 1C] Figure 1C shows the results obtained by subjecting R3s to comprehensive transcriptomic analysis and up-stream analysis.
[Figure 2] Figure 2 is figures showing the effect of thiostrepton on the osteoclast differentiation of R3s (in vitro). Photographs indicate that osteoclast differentiation is suppressed by thiostrepton administration (reds show osteoclasts, and greens show precursor cells). The vertical axis of the graph shows the number of nuclei contained in osteoclasts in one visual field with cells having three or more nuclei defined as osteoclasts.
[Figure 3A] Figure 3A is a graph showing the results obtained by administering thiostrepton to arthritis model mice (CIA model) and evaluating the effects thereof using arthritis scores.
[Figure 3B] Figure 3B shows the results obtained by administering thiostrepton to arthritis model mice (CIA model) similarly and evaluating the osteoclastic levels of articulations using osteoclastic scores imaged by micro-CT. "Thio" indicates thiostrepton. The photographs show images of bone tissues photographed by micro-CT, and the vertical axis of the graph is the erosion score, and shows the level of bone destruction.
[Figure 4A] Figure 4A is a figure showing a protocol for causing arthritis in a FoxM1 knockout mouse induced by tamoxifen administration. Figure 4A indicates that 2 mg of tamoxifen is intraperitoneally administered for 5 days, 5 mg of a CAIA antibody is intravenously injected, and arthritis is evaluated 2 weeks after.
[Figure 4B] Arthritis was caused in FoxM1 knockout mice produced according to the protocol shown in Figure 4A, and the osteoclastic levels were confirmed. Photographs are images of bone tissues photographed by micro-CT, and the vertical axis of the graph is the erosion score, and shows the level of bone destruction.
[Figure 5A] Figure 5A is the results obtained by comparing samples of blood, synovial fluid, and articular tissue of a rheumatoid arthritis patient (human) by flow cytometry. CX3CR1-positive cells of HLA-DRhi are observed in the synovial fluid and the articular tissue.
[Figure 5B] Figure 5B shows the effect of thiostrepton on the osteoclast differentiation of CX3CR1-positive cell fractions of HLA-DRhi of the rheumatoid arthritis patient shown in Figure 5A. The photographs indicate that the osteoclast differentiation is inhibited by thiostrepton administration. The vertical axis of the graph shows the number of nuclei contained in osteoclasts in one visual field when cells having three or more nuclei are defined as osteoclasts.

### Description of Embodiments

A FoxM1 inhibitor used in the present invention is preferably an inhibitor of FoxM1 activity comprising a low molecular weight compound such as thiostrepton or RCM-1 (the Non Patent Literature 2).

Meanwhile, a compound which is able to suppress the expression of FoxM1 can also be used as a FoxM1 inhibitor of the present invention, and, for example, all the compounds that inhibit the transcription of its gene, the maturation of the RNA, the translation of the mRNA, the posttranslational modification of its protein, and the like are relevant as such. A splicing modifier (refer to the Patent Literature 1), siRNA (refer to the Patent Literature 2), and the like of the FoxM1 gene can be specifically mentioned.

A therapeutic agent for arthritis containing the FoxMl inhibitor as an active ingredient of the present invention can be widely used for treatment and/or prevention of illnesses accompanied with the inflammation of articulations. For example, rheumatoid arthritis, spondylarthritis, histiocytosis, and the like are mentioned as target diseases. The therapeutic agent is however preferably used for treatment and/or prevention of rheumatoid arthritis.

The FoxM1 inhibitor of the present invention can be prepared into the form of a conventional pharmaceutical preparation (medicinal composition) suitable for oral administration, parenteral administration, or local administration.

The preparation for oral administration includes solid agents such as tablets, granules, powders, and capsules and liquid preparations such as syrups. These preparations can be prepared by conventional methods. The solid agents can be prepared using conventional pharmaceutical carriers such as lactose; starch such as cornstarch; crystalline cellulose such as microcrystalline cellulose; hydroxypropylcellulose; calcium carboxymethyl cellulose; talc; and magnesium stearate. The capsules can be prepared by encapsulating the thus prepared granules or powders. The syrups can be prepared by dissolving or suspending the FoxM1 inhibitor in a solution containing sucrose, carboxymethyl cellulose, or the like.

The preparation for parenteral administration includes infusions such as drip infusion. Injectable preparations can also be prepared by conventional methods, and can be optionally incorporated into an isotonizing agent (for example, mannitol, sodium chloride, glucose, sorbitol, glycerol, xylitol, fructose, maltose, or mannose), a stabilizer (for example, sodium sulfite or albumin), and an antiseptic (for example, benzyl alcohol, methyl p-oxybenzoate).

Dosage forms for local or percutaneous administration include ointments, pastes, creams, lotions, gels, powdered drug, solutions, sprays, inhalants, or patches. The FoxM1 inhibitor which is an active ingredient is mixed with a pharmaceutically acceptable carrier and a required preservative or a buffer solution which can be required under the aseptic condition.

Although the dosage of the FoxM1 inhibitor of the present invention can be changed depending on the compound having the inhibiting activity; the type and the severity of disease; the age, the sex, and the weight of a patient; and the dosage form, the dosage is usually in the range of 1 mg to 1,000 mg per day in an adult. It can be divided into 1, 2, or 3 portions, and the portions can be administered by the oral route or the parenteral route.

According to the present invention, a method for treating arthritis using the FoxM1 inhibitor is provided. In this case, the FoxM1 inhibitor can be used alone or in combination with one or more other therapeutic agents. The FoxM1 inhibitor can be administered in combination with least one therapeutic drug for arthritis such as a disease modifying anti-rheumatic drug (DMARD), a pain control drug, a steroid, a non-steroid anti-inflammatory drug (NSAID), a cytokine antagonist, a bone assimilation agent, an antiresorptive agent, and a combination thereof (a dual therapy or a triple therapy) as a drug used in combination. When the FoxM1 inhibitor is administered in combination with one or more additive drugs, the FoxM1 inhibitor can be administered simultaneously with other drugs or sequentially. In the case of the sequential administration, doctors determine a suitable order in which the FoxM1 inhibitor is administered in combination with other drugs.

As DMARDs used in combination with the FoxM1 inhibitor, methotrexate, antimalarial drugs (for example, hydroxychloroquine and chloroquine), sulfasalazine, leflunomide, azathioprine, cyclosporin, gold salts, minocycline, cyclophosphamide, D-penicillamine, minocycline, auranofin, tacrolimus, Myochrysine, chlorambucil, and the like are mentioned; as steroids, prednisolone, prednisone, dexamethasone, cortisol, cortisone, hydrocortisone, methylprednisolone, betamethasone, triamcinolone, beclomethasone, fludrocortisone, deoxycorticosterone, aldosterone, and the like are mentioned; and as pain control drugs or non-steroid anti-inflammatory drugs (NSAIDs), lumiracoxib, ibuprofen, fenoprofen, ketoprofen, flurbiprofen, oxaprozin, indomethacin, sulindac, etodolac, ketorolac, nabumetone, aspirin, naproxen, valdecoxib, etoricoxib, rofecoxib, acetominophen, celecoxib, diclofenac, tramadol, piroxicam, meloxicam, tenoxicam, droxicam, lornoxicam, isoxicam, mefenamic acid, meclofenamic acid, flufenamic acid, tolfenamic, valdecoxib, parecoxib, etodolac, indomethacin, aspirin, ibuprofen, firocoxib, and the like are mentioned. However, the drugs used in combination with the FoxM1 inhibitor are not limited to these.

Although the present invention will be described specifically by showing the Examples, these Examples never limit the present invention.

### Example 1

### Test Example 1 Expression of FoxM1 in osteoclast precursor cells

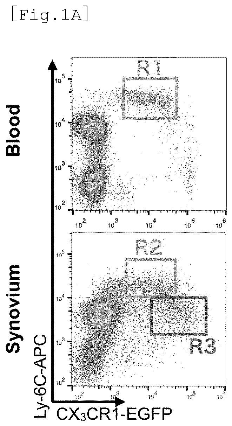

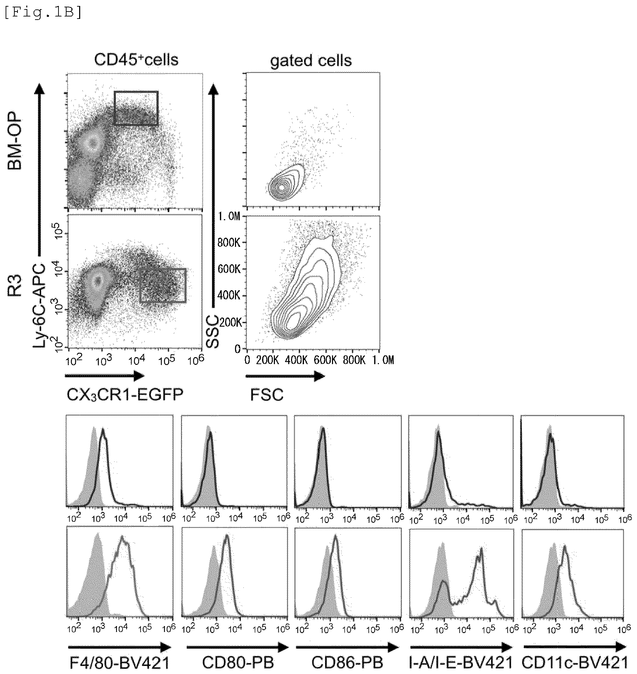

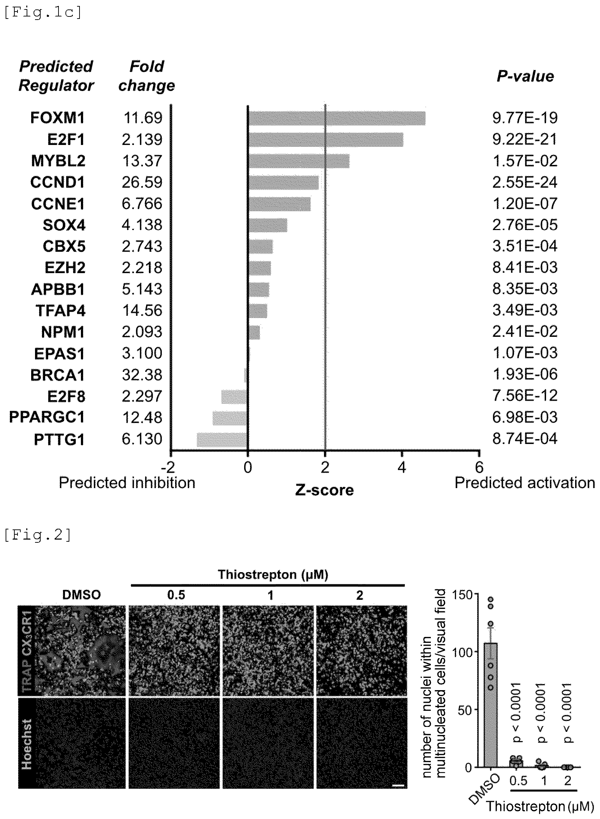

A) CD45-positive cells of blood and a synovial membrane of a collagen-induced arthritis model mouse (CIA model) were selected by flow cytometry, and the cell fractions were identified with the fluorescence of CX3CR1-EGFP and Ly6C-APC. A CX3CR11owLy6Chi fraction in blood was defined as R1, a CX3CR11owLy6Chi fraction in an inflammatory synovial membrane was defined as R2, and a CX3CR1hiLy6Cint fraction was defined as R3 (Figure 1A).
B) The R3 fraction was analyzed by flow cytometry, so that CD80, CD86, I-A/I-E, and CD11c were expressed (Figure 1B) unlike fractions previously reported to be osteoclast precursor cells (BM-OP) in bone marrow.
C) RNA-sequence analysis was performed to comprehensively analyze the expression situations of mRNA of the R1, R2, and R3 fractions shown in the above-mentioned A) (Illumina HiSeq 2500 platform in 75-base single-end mode). When transcription factors increasing in the R3 were listed and subjected to up-stream analysis, FoxM1 was ranked first, and FoxM1 was shown as a control factor of the present cells (Figure 1C).

### Test Example 2 Suppression of differentiation into osteoclasts (in vitro)

The R3 cells were collected using flow cytometry (cell sorter SH800, Sony Corporation) and cultured with 10 ng/ml M-CSF, 100 ng/ml RANKL, 0.5, 1, or 2 µM thiostrepton (Sigma-Aldrich Japan) which is a FoxM1 inhibitory drug added thereto on a 96-well plate, the capability to differentiate into osteoclasts was remarkably suppressed in thiostrepton treatment groups (Figure 2).

### Test Example 3 Effect in model mouse (in vivo)

Collagen-induced arthritis (CIA) mice were divided into three groups, and 20 mg/kg or 50 mg/kg of thiostrepton or a base (contrast) was intraperitoneally administered every other day from the twenty-first day. Methods for producing and evaluating CIA mice are as follows.

Arthritis was caused using 8- to 10-week-old DBA-1/J mice. Chicken type II collagen was dissolved in a 0.05 M acetic acid solution and rotated at 4°C all night to make a solution at a concentration of 4.0 mg/ml, and the solution was mixed with the same volume of Freund's complete adjuvant. On the first day, 100 µl of the emulsion was injected into the tail of each of the above-mentioned DBA-1/J mice for immunization, and the administration was repeated in the same way on the twenty-first day. The severity of arthritis was evaluated in accordance with a semi-quantitative score method which consists of an established 5-point scale. The scale is as follows: 0: no swelling, 1: slight swelling only in the heel or the tarsal bones, 2: slight swelling from the heel to the tarsal bones, 3: considerable swelling from the heel to the metatarsal articulation, and 4: severe swelling spreading over an area including the heel, the foot, and the toes.

The cumulative score of the four limbs of each mouse (the maximum is 16) is used as an arthritis score [refer to Brand, D. D., Latham, K. A. & Rosloniec, E. F. Collagen-induced arthritis. Nat. Protoc. 2, 1269-1275 (2007)].

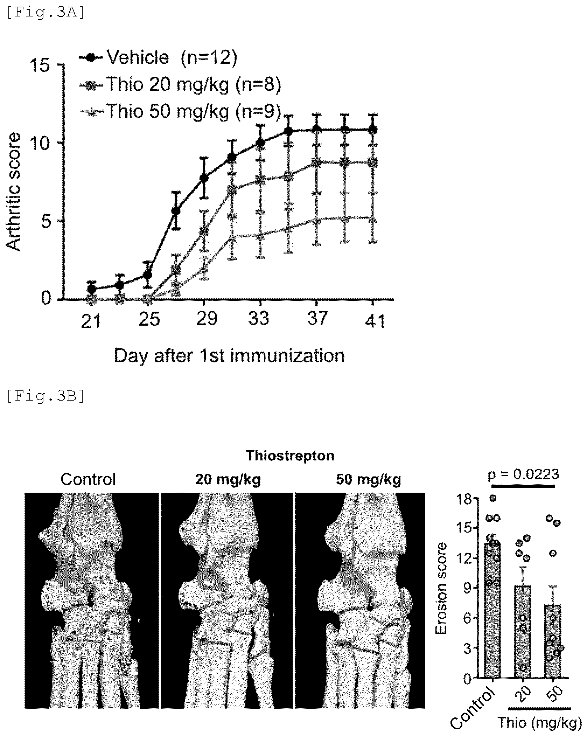

Image analysis using micro-CT was performed. The osteoclastic score was determined with reference to previous study (refer to O'Brien, Arthritis Rheumatol. 2016). The above-mentioned arthritis score and the osteoclastic score imaged by micro-CT decreased significantly as compared with the control group (Figures 3A and 3B).

### Test Example 4 Evaluation in knockout mouse

Since FoxM1 knockout mice die during the fetal period, a CAIA model was evaluated using mice in which FoxM1 was knocked out with tamoxifen specifically to time (RosaERT2Cre;FoxM1fl/fl). Although, in the conventional CIA model, arthritis was caused in the lineage DBA1/J, arthritis was hardly caused in the lineage of knockout mice called B6. Therefore, the CAIA model in which arthritis was steadily caused also in the B6 was used.

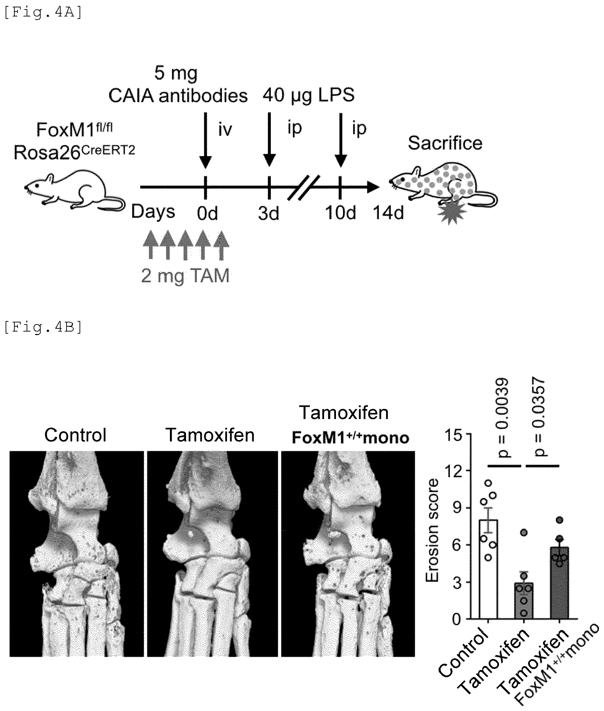

The knockout model was produced in accordance with the protocol shown in Figure 4A. That is, 5 mg of 5-clone Arthrogen-CAIA antibodies (Chondrex, Inc.) was iv administered on the first day, 40 µg of LPS was intraperitoneally administered on the third and tenth days, and arthritis was caused. The severity of arthritis was evaluated by the same semi-quantitative method as that of the above-mentioned CIA model. These arthritis model mice were divided into a control group, a group to which 2 mg of tamoxifen was intraperitoneally administered for 5 days, and a group to which monocytes in which FoxM1 was not knocked out was further adoptively transferred after tamoxifen administration. The osteoclastic scores were compared on the fourteenth day.

Then, the osteoclastic score was significantly suppressed in the tamoxifen treatment group as compared with the control group. When monocytes in which FoxM1 was not knocked out was further adoptively transferred thereto, the bone destruction was exacerbated (Figure 4B) .

### Test Example 5

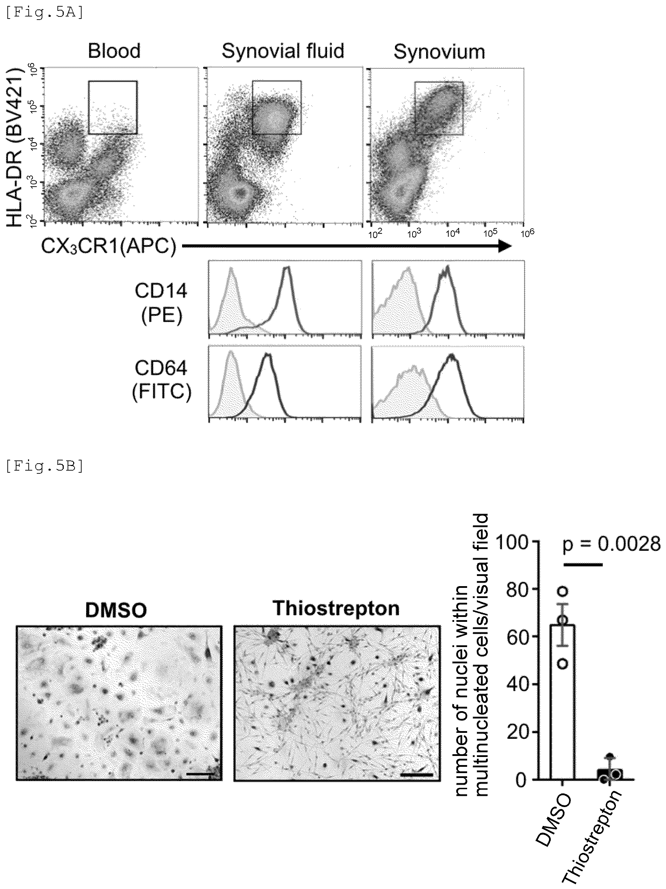

When samples of blood, synovial fluid, and an articulation tissue of a rheumatoid arthritis patient (human) were evaluated using flow cytometry, CX3CR1-positive HLA-DRhi cells were observed in the synovial fluid and the articulation tissue in the same way as the R3 of the Test 1 (I-A/I-E in mice corresponds to human HLA-DR, and it was shown in Figure 1B that HLA-DR was highly expressed also in the R3). When these cells were collected by flow cytometry and cultured on a 96-well plate with 50 ng/ml M-CSF, 100 ng/ml RANKL, and 0.5 µM thiostrepton added. The differentiation into osteoclasts was significantly suppressed by thiostrepton administration (Figures 5A and 5B).

### Industrial Applicability

Since a therapeutic agent for arthritis without the risk of contracting severe infectious diseases is produced by incorporating an inhibitor of FoxM1 as an active ingredient, the present invention can be utilized industrially.

## Claims

1. A therapeutic agent for arthritis, comprising an inhibitor of FoxM1 as an active ingredient.

2. The therapeutic agent according to claim 1, wherein the inhibitor of FoxM1 is an inhibitor of FoxM1 activity.

3. The therapeutic agent for arthritis according to claim 2, wherein the inhibitor of the FoxM1 activity is thiostrepton.

4. The therapeutic agent for arthritis according to claim 2, wherein the inhibitor of the FoxM1 activity is RCM-1.

5. The therapeutic agent according to any one of claims 1 to 4, wherein arthritis is rheumatoid arthritis.

6. The therapeutic agent according to any one of claims 1 to 5, wherein the therapeutic agent is a preparation for oral administration.

7. A method for treating arthritis or rheumatoid arthritis, comprising administering a therapeutically effective amount of the therapeutic agent according to any one of claims 1 to 6 to a patient needing treatment.

8. Use of the FoxM1 inhibitor according to any one of claims 1 to 4 for producing a therapeutic drug for arthritis or rheumatoid arthritis.
